(19) Europäisches Patentamt
European Patent Office
Office européen des brevets

(11) Veröffentlichungsnummer: **0 027 529**
**A1**

(12) **EUROPÄISCHE PATENTANMELDUNG**

(21) Anmeldenummer: **80105269.7**

(22) Anmeldetag: **04.09.80**

(51) Int. Cl.³: **C 07 D 417/04**
C 07 D 311/16, C 07 D 405/04
C 09 B 57/02, C 08 K 5/47

(30) Priorität: **15.09.79 DE 2937422**

(43) Veröffentlichungstag der Anmeldung:
**29.04.81 Patentblatt 81/17**

(84) Benannte Vertragsstaaten:
**AT BE CH DE FR GB IT LI LU NL SE**

(71) Anmelder: **BASF Aktiengesellschaft**
**Carl-Bosch-Strasse 38**
**D-6700 Ludwigshafen(DE)**

(72) Erfinder: **Seybold, Guenther, Dr.**
**Otto-Dill-Strasse 32**
**D-6700 Ludwigshafen(DE)**

(72) Erfinder: **Vamvakaris, Christos, Dr.**
**Riedweg 6**
**D-6701 Kallstadt(DE)**

(54) **Cumarin-Derivate und ihre Verwendung zum Färben synthetischer Fasern oder von Kunststoffen.**

(57) Die Erfinding betrifft Verbindungen der allgemeinen Formel I

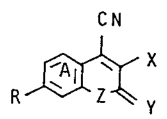

1

in der

R Wasserstoff, gegebenenfalls substituiertes Alkyl, Cycloalkyl, Alkoxy, Phenoxy, Hetaryloxy, Alkylmercapto, Arylmercapto, Aryl, Acylamino, Hydrazino, Hetarylamino, Acyloxy, Alkylaminocarbonyloxy oder Arylaminocarbonyloxy, Halogen, Hydroxyl, Nitro, Sulfonamido, Cyan oder einen in o-Stellung ankonensierten Ring,

X einen elektronenziehenden Rest,

Y NH, $NR^1$, O, S oder 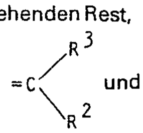 und

Z O, NH, $NR^1$ oder S bedeuten wobei

$R^1$ gegebenenfalls substituiertes Alkyl, Cycloalkyl, Aryl oder Acyl und

$R^2$ und $R^3$ unabhängig voneinander Cyan, Carbonester, gegebenenfalls substituiertes Carbamoyl oder Hetaryl sind und wobei der Ring A noch durch einen weiteren Rest R substituiert sein kann.

Die Verbindung der Formel I eignen sich zum Färben synthetischer Fasern und von Kunststoffen in der Masse.

EP 0 027 529 A1

## Heterocyclische Verbindungen

Die Erfindung betrifft Verbindungen der allgemeinen Formel I

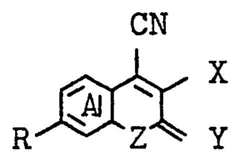

I

in der

R Wasserstoff, gegebenenfalls substituiertes Alkyl, Cycloalkyl, Alkoxy, Phenoxy, Hetaryloxy, Alkylmercapto, Arylmercapto, Aryl, Acylamino, Hydrazino, Hetarylamino, Acyloxy, Alkylaminocarbonyloxy oder Arylaminocarbonyloxy, Halogen, Hydroxyl, Nitro, Sulfonamido, Cyan oder einen in o-Stellung ankonensierten Ring,

X einen elektronenziehenden Rest,

Y NH, $NR^1$, O, S oder 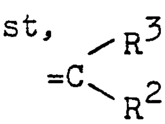 und

Z O, NH, $NR^1$ oder S bedeuten, wobei

$R^1$ gegebenenfalls substituiertes Alkyl, Cycloalkyl, Aryl oder Acyl und

$R^2$ und $R^3$ unabhängig voneinander Cyan, Carbonester, gegebenenfalls substituiertes Carbamoyl oder Hetaryl sind und wobei der Ring A noch durch einen weiteren Rest R substituiert sein kann.

Bg/BL

Reste R sind neben den bereits genannten z. B. $C_1$- bis $C_4$-Alkyl, Cyclohexyl, Benzyl, $C_1$- bis $C_{10}$-Alkoxy, $C_1$- bis $C_{10}$-Hydroxy-, Phenoxy- oder Alkoxypolyalkoxy, durch Chlor, Brom, Methyl, Äthyl, Methoxy oder Äthoxy substituiertes Phenoxy, $C_1$- bis $C_4$-Alkylmercapto, durch Chlor, Brom, Methyl oder Methoxy substituiertes Phenylmercapto, Phenyl, gegebenenfalls am N durch $C_1$- bis $C_8$-Alkyl substituiertes $C_1$- bis $C_8$-Alkanoylamino oder Benzoylamino, $C_1$- bis $C_8$-Alkanoyloxy, Benzoyloxy, $C_1$- bis $C_4$-Alkylaminocarbonyloxy, gegebenenfalls durch Chlor oder Methyl substituiertes Phenylaminocarbonyloxy, Chlor oder Brom.

Einzelne Reste R sind neben den bereits genannten beispielsweise:

$CH_3$-, $C_2H_5$-, $C_3H_7$-, $CH_3O$-, $C_2H_5O$-, $C_3H_7O$-, $NCC_2H_4$-O-, $HOC_2H_4O$-, $CH_3OOC$-$C_2H_4O$-, $(C_2H_5)_2N$-$C_2H_4O$-, $(C_2H_5)_2N$-$C_3H_6O$-, $C_6H_5CH_2OC_2H_4$-O-, $C_6H_5O$-$C_2H_4O$-, $C_2H_5OOC$-$C_2H_4O$-, $CH_3OC_2H_4O$-, $C_2H_5OC_2H_4O$-, $C_4H_9OC_2H_4O$-, HO-$C_2H_4OC_2H_4O$-, $ClCH_2CHOHCH_2$-, $C_6H_5OC_2H_4OC_3H_6O$-, $CH_3OC_2H_4OC_2H_4O$-, $C_6H_5O$-, $CH_3S$-, $C_2H_5S$-, $C_4H_9S$-, $C_6H_5S$-, Cl-$C_6H_4S$-, $BrC_6H_4S$-,

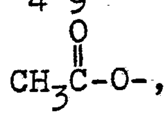, 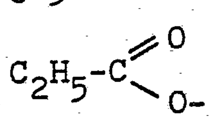, 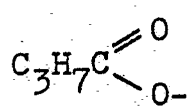, $C_6H_{13}COO$-, 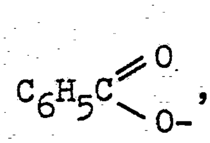,

Cl, Br, $C_{17}H_{35}COO$-, 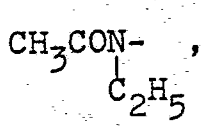, 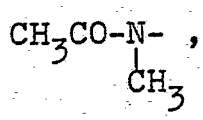, 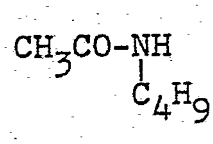

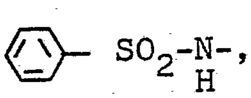, 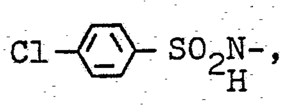, 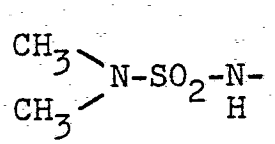

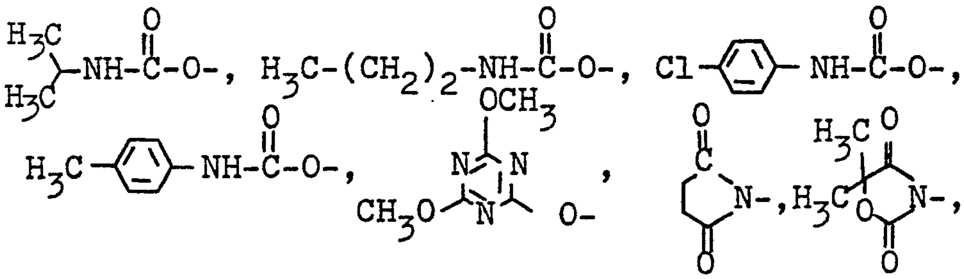

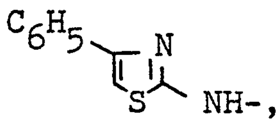, 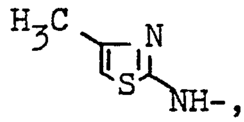, 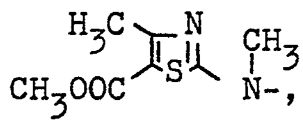,

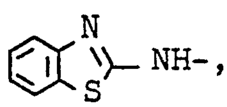, 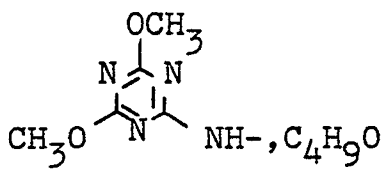, 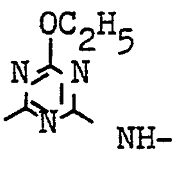 oder

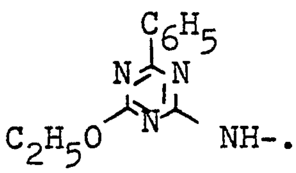.

Als elektronenziehende Reste X kommen z. B. in Betracht:

1) Cyan-, $C_1$- bis $C_{20}$-Alkoxycarbonyl oder gegebenenfalls substituiertes Carbonamid.

2) der Rest 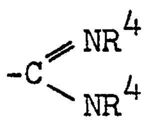, wobei $R^4$ unabhängig voneinander einen gegebenenfalls substituierten Alkyl-, Cycloalkyl-, Aralkyl- oder Arylrest bedeutet.

3) der Rest 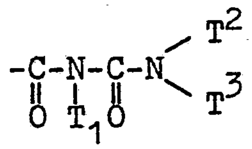 wobei $T^1$, $T^2$, $T^3$ unabhängig voneinander Wasserstoff, einen Acyl- oder einen gegebenenfalls substituierten Alkyl- oder Phenylrest bedeuten.

4) der Rest $-SO_2T^4$ wobei $T^4$ gegebenenfalls substituiertes Alkyl, Phenyl oder Heteroaryl bedeutet.

5) der Rest 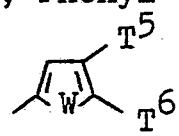 wobei W einen zweiwertigen Rest der Formel -O-, -S- oder -CH=CH- und $T^5$ und $T^6$ unabhängig voneinander Wasserstoff, Cyan, Alkoxycarbonyl, Carbonamid, Halogen, Alkylsulfonyl, gegebenenfalls substituiertes Sulfonamid oder Nitro bedeuten oder zusammen auch einen gegebenenfalls substituierten aromatischen oder heteroaromatischen Ring bilden.

6) der Rest der allgemeinen Formel A

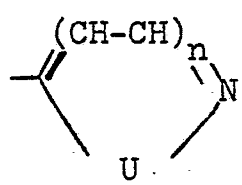 A,

wobei n = 0 oder 1 und U ein zweiwertiger Rest ist, der mit dem N einen 5- oder 6-gliedrigen Ring bildet. Insbesondere bedeutet der Rest der Formel A einen gegebenenfalls substituierten oder anellierten Pyrazol-, Imidazol-, Thiazol-, Oxazol-, 1,2,4-Triazol-, 1,3,4-Oxdiazol-, 1,3,4-Thiadiazol-, Chinoxalon-, Chinazolon-, Benzimidazol-, Benzoxazol-, Benzthiazol-, Pyridin-, Chinolin- oder Pyrimidinring.

Als Reste $R^1$ kommen im Rahmen der allgemeinen Definition die bereits für R genannten Gruppen in Betracht.

Reste $R^2$ und $R^3$ sind unabhängig voneinander im einzelnen beispielsweise:

Cyan, Carbalkoxy, Carbonamid, gegebenenfalls substituiertes Phenyl oder Heteroaryl.

Einzelne Reste $R^1$ und $R^2$ sind neben den bereits genannten beispielsweise:

$-COOC_2H_5$, $-COOC_4H_9$, $-COOC_2H_4OC_2H_5$, $-CON(C_2H_5)_2$, 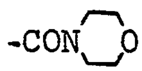,

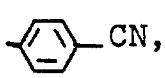, 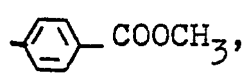, 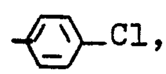, 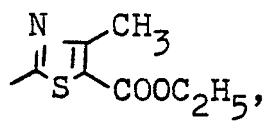,

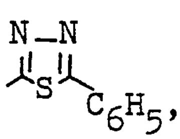, 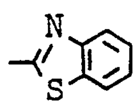 oder 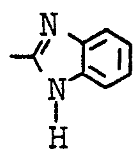 .

Zur Herstellung der Verbindungen der Formel I kann man an Verbindungen der Formel II

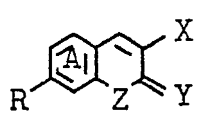 II

HCN anlagern und die so erhaltenen Produkte zu den Verbindungen der Formel I oxidieren.

Einzelheiten der Herstellung können den Beispielen entnommen werden, in denen sich Angaben über Teile und Prozente, sofern nicht anders vermerkt, auf das Gewicht beziehen.

Von besonderer Bedeutung sind Verbindungen der Formel I bei denen

R Alkyl, Alkoxy, Acylamino, Acyloxy oder Alkylaminocarbonyloxy mit jeweils 1 bis 4 C-Atomen oder Triazinyloxy- und

X einen Rest der Formel

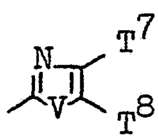 oder 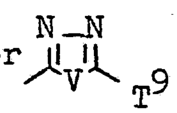

bedeuten, wobei

V ein Rest der Formel -S-, -O-, -NH oder N-$T^{10}$,

$T^7$ und $T^8$ unabhängig voneinander Wasserstoff, gegebenenfalls substituiertes Alkyl oder Phenyl, gegebenenfalls substituiertes Alkyl- oder Phenylmercapto, Cyan, Carbamoyl oder Carbonester oder ggf. im Ring durch Chlor, Brom, Methyl, Äthyl, Methoxy oder Äthoxy substituiertes Benzyl,

$T^8$ darüber hinaus noch $C_2$- $C_4$-Alkanoyl, gegebenenfalls durch Chlor, Methyl, Methoxy oder Äthoxy substituiertes Benzoyl, $C_1$- bis $C_6$-Alkylsulfonyl oder gegebenenfalls durch Chlor, Brom, Methyl, Methoxy, Äthoxy, Amino, $C_1$ - $C_4$ Alkanoylamino oder

Benzoylamino substituiertes Phenylsulfonyl,

$T^7$ und $T^8$ zusammen einen gesättigten, gegebenenfalls Sauerstoff oder Schwefel enthaltenden 5- oder 6-gliedrigen gesättigten Ring oder einen gegebenenfalls ein- oder mehrfach durch $C_1 - C_4$ Alkyl, Phenyl, Chlor, Brom, Alkoxy oder Alkylsulfonyl substituierten Benzring,

$T^9$ gegebenenfalls substituiertes Alkyl, Alkenyl, Cycloalkyl, Aralkyl, Aryl oder Heteroaryl oder ein Rest der Formel O-B, -SB, $-SO_2B$ oder

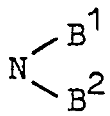 und

$T^{10}$ gegebenenfalls substituiertes Alkyl oder Aryl sind, wobei

B einen gegebenenfalls substituierten Alkyl-, Alkenyl-, Cycloalkyl-, Aralkyl-, Aryl oder Heteroarylrest

$B^1$ und $B^2$ unabhängig voneinander Wasserstoff, gegebenenfalls substituiertes Alkyl, Cycloalkyl, Aralkyl oder Aryl oder

$B^1$ und $B^2$ zusammen mit dem Stickstoff einen 5- oder 6-gliedrigen heterocyclischen Ring bedeuten.

Y ist vorzugsweise O oder NH und

Z Sauerstoff.

Technisch besonders wertvoll sind Verbindungen der Formel I a

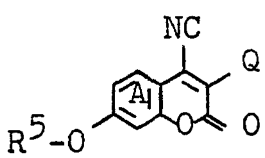

I a

bei denen beispielsweise

$R^5$ H, $C_1$ - $C_4$-Alkyl, $C_1$ - $C_{14}$-Phenoxy- oder Alkoxypolyalkoxyalkyl, durch Chlor, Brom, Methyl, Äthyl, Methoxy oder Äthoxy substituiertes Phenyl oder $C_1$ - $C_6$-Acyl und

Q einen Rest der Formel

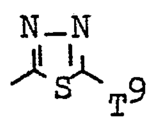

oder

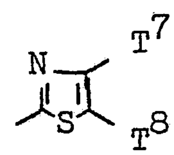

bedeuten, wobei

$T^7$, $T^8$ und $T^9$ die bereits angegebene Bedeutung besitzen.

Darüber hinaus kann $R^5$ mit dem Sauerstoff einen in ortho-Stellung zum Sauerstoff ankondensierten gesättigten oder ungesättigten Ring bilden.

Die Verbindungen der Formel I fluoreszieren und ergeben auf Textilmaterial aus synthetischen Fasern, insbesondere Polyestern, brillante, farbstarke Färbungen mit guten bis sehr guten Echtheiten, von denen die Lichtechtheiten hervorzuheben sind.

Die Verbindungen der Formel I eignen sich ferner zum Färben von Kunststoffen in der Masse, wobei die Kunststoffe z. B. in Sonnenkollektoren verwendet werden können.

Beispiel 1

Zu 5,5 Teilen 3-(4'-Cyan-phenyl)-7-methoxy-cumarin in 100 Volumenteile Dimethylformamid läßt man 5,4 Teile einer 6-normalen NaCN Lösung bei Raumtemperatur zulaufen. Nach beendeter Addition wird das Reaktionsgemisch noch weitere 2 Stunden gerührt.

Man kühlt dann auf 0 - 5 °C ab, setzt portionsweise 10 Teile Bleitetraacetat vorsichtig zu und rührt weitere 60 Minuten unter Kühlung nach. Das Reaktionsgemisch wird in Eiswasser eingerührt, das Reaktionsprodukt abgesaugt und mit viel Wasser gewaschen. Man erhält 6 Teile 3-(4'-Cyanphenyl)-4-cyan-7-methoxycumarin vom Schmelzpunkt 230 - 240 °C (aus Äthanol).

Beispiel 2

Zu 6 Teilen 3-Cyan-7-methoxycumarin in 60 Volumenteilen Dimethylformamid läßt man 7,8 Teile einer 6-n NaCN-Lösung so zulaufen, daß die Temperatur nicht über 30 °C ansteigt. Nach beendeter Addition wird das Reaktionsgemisch noch 2 Stunden bei Raumtemperatur gerührt. Man kühlt dann auf 0 - 5 °C ab, setzt portionsweise 10 Teile Bleitetraacetat vorsichtig zu und rührt weitere 60 Minuten unter Kühlung nach. Das Reaktionsgemisch wird dann in Eiswasser eingerührt, das Reaktionsprodukt abgesaugt und mit viel Wasser gewaschen. Man erhält 6,9 Teile 3,4-Dicyan-7-methoxycumarin vom Schmelzpunkt 200 - 210 °C (unter Zersetzung).

Verwendet man an Stelle von Bleitetraacetat andere Oxydationsmittel wie Natrium- oder Kaliumpersulfat, Chloranil, Wasserstoffperoxid oder Brom, erhält man vergleichbare Ergebnisse.

Beispiel 3

3,5 Teile der Verbindung

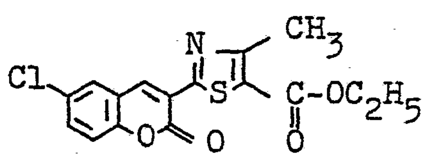

werden in 50 Volumenteilen Dimethylformamid mit 2,7 Teilen einer 6-n NaCN Lösung tropfenweise versetzt, so daß die Temperatur nicht über 30 °C ansteigt. Nach beendeter Addition wird die Reaktionsmischung noch weitere 2 Std. bei Raumtemperatur nachgerührt. Dann kühlt man auf 0 - 5 °C ab und setzt vorsichtig 8 Teile Bleitetraacetat zu, wobei man die Temperatur nicht über 5 °C ansteigen läßt und rührt noch eine Stunde bei 0 - 5 °C nach.

Das Reaktionsprodukt wird in Eiswasser eingerührt, abgesaugt und mit Wasser gewaschen. Man erhält 5,2 Teile der Verbindung

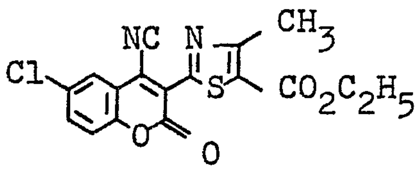

vom Schmelzpunkt 242 - 250 °C.

Beispiel 4

6,9 Teile der Verbindung

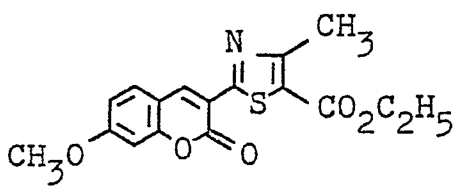

werden in 100 Volumenteilen Dimethylformamid mit 5,4 Teilen einer 6-n NaCN Lösung bei Raumtemperatur versetzt. Das Reaktionsgemisch wird 1 Stunde bei Raumtemperatur nachgerührt. Danach wird bei 0 - 5 °C portionsweise mit 10 Teilen Bleitetraacetat vorsichtig versetzt und 1 Stunde nachgerührt. Das Reaktionsgemisch wird in Eiswasser gegossen und der ausgefallene Farbstoff filtriert und mit viel Wasser gewaschen. Man erhält 6,9 Teile der Verbindung

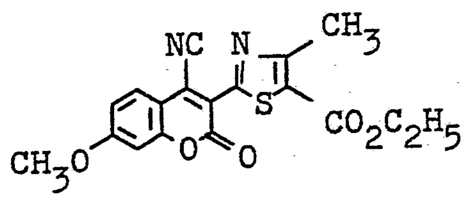

vom Schmelzpunkt 256 - 257 °C.

Der Farbstoff färbt Polyesterfasern in fluoreszierenden grünstichig gelben Tönen mit sehr guten Echtheiten.

Analog erhält man die in der Tabelle 1 aufgeführten Verbindungen.

Tabelle 1

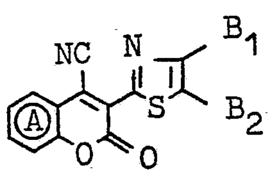

| Bsp. | 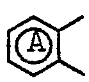 | $B_1$ | $B_2$ |
|---|---|---|---|
| 5 | 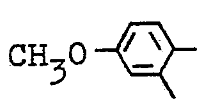 | $CH_3$ | 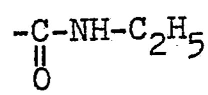 |
| 6 | " | " | 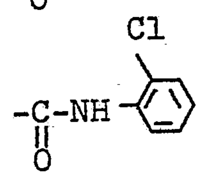 |

| Bsp. | 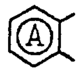 | $B_1$ | $B_2$ |
|---|---|---|---|
| 7 | 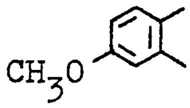 | $CH_3$ | $C_6H_5$ |
| 8 | 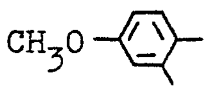 | " | 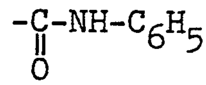 |
| 9 | " | " | 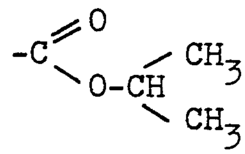 |
| 10 | " | " | $C_6H_5$ |
| 11 | " | " | 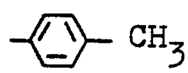 |
| 12 | 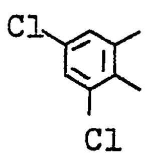 | " | " |
| 13 | " | $C_6H_5$ | $CH_3$ |
| 14 | 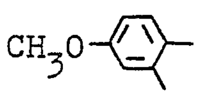 | $CH_3$ | $CO_2CH_3$ |
| 15 | " | " | $CO_2C_2H_5$ |
| 16 | " | " | 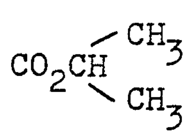 |

| Bsp. | 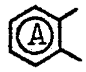 | $B_1$ | $B_2$ |
|---|---|---|---|
| 17 | 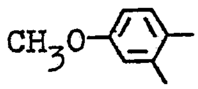 | $CH_3$ | 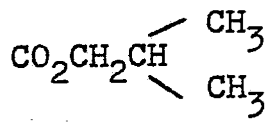 |
| 18 | " | " | $CO_2-C_2H_4-OCH_3$ |
| 19 | " | " | $CO_2C_2H_4-OC_4H_9$ |
| 20 | 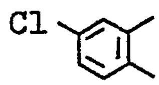 | " | " |
| 21 | 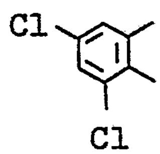 | " | " |
| 22 | 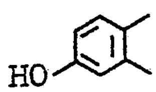 | " | $CO_2C_2H_5$ |
| 23 | 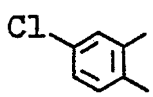 | " | " |
| 24 | " | $C_6H_5$ | $CH_3$ |
| 25 | 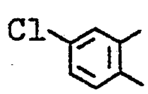 | $C_6H_4CH_3(p)$ | " |
| 26 | 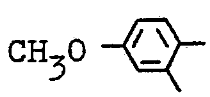 | " | " |
| 27 | " | $CH_3$ | $COCH_3$ |

| Bsp. | 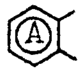 | $B_1$ | $B_2$ |
|---|---|---|---|
| 28 | 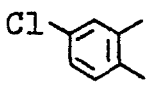 | $CH_3$ | $COCH_3$ |
| 29 | 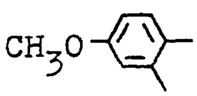 | " | $COC_6H_5$ |
| 30 | " | " | 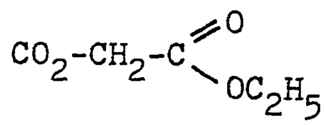 |
| 31 | " | " | 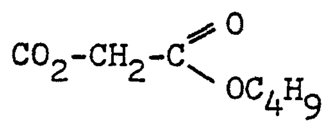 |

Beispiel 32

5,5 Teile der Verbindung

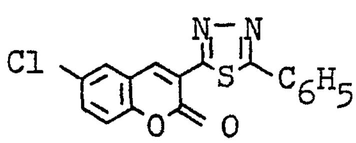

werden bei Raumtemperatur in 60 Volumenteilen Dimethylformamid mit 4,3 Teilen einer 6-n NaCN Lösung tropfenweise versetzt. Nach beendeter Addition wird das Reaktionsgemisch noch weitere 2 Std. bei Raumtemperatur gerührt. Dann wird die Reaktionslösung abgekühlt und bei 0 - 5 °C mit 8 Teilen Bleitetraacetat vorsichtig versetzt. Die Reaktionsmischung wird anschließend noch

1 Stunde bei 0 - 5 °C nachgerührt und dann auf Eiswasser gegossen. Der ausgefallene Feststoff wird abgesaugt und mit viel Wasser gewaschen. Man erhält 5,2 Teile der Verbindung

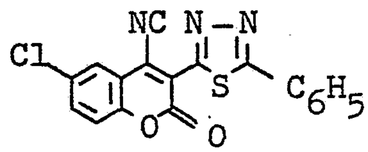

vom Schmelzpunkt über 300 °C.

Beispiel 33

5,4 Teile der Verbindung

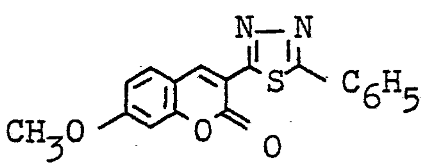

werden bei Raumtemperatur in 60 Volumenteilen Dimethylformamid mit 4,3 Teilen einer 6-n NaCN Lösung tropfenweise versetzt. Nach beendeter Addition wird das Reaktionsgemisch noch weitere 2 Std. bei Raumtemperatur gerührt. Die Reaktionslösung wird dann abgekühlt und bei 0 - 5 °C portionsweise mit 8 Teilen Bleitetraacetat versetzt. Man rührt noch 1 Stunde nach und gießt das Reaktionsgemisch in Wasser. Der ausgefallene Farbstoff wird abgesaugt und mit Wasser gewaschen. Man erhält 6,4 Teile der Verbindung

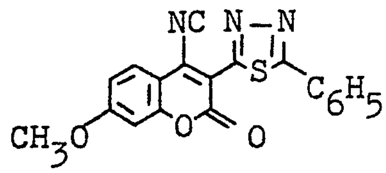

vom Schmelzpunkt 255 - 258 °C. Der Farbstoff färbt Polyesterfasern nach den üblichen Färbeverfahren in grünstichig-gelben fluoreszierenden Tönen mit sehr guten Echtheiten.

Analog lassen sich die in der Tabelle 2 aufgeführten Verbindungen herstellen.

Tabelle 2

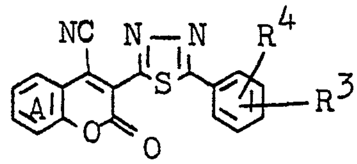

| Bsp. | 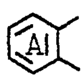 | $R^3$ | $R^4$ |
|---|---|---|---|
| 34 | 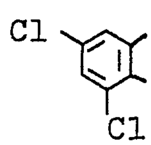 | H | H |

| Bsp. | 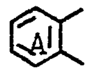 | $R^3$ | $R^4$ |
|---|---|---|---|
| 35 | 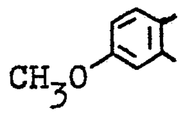 | 4-Cl | H |
| 36 | " | 2-Cl | H |
| 37 | 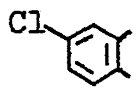 | 4-Cl | H |
| 38 | 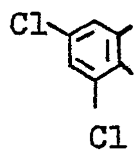 | 2-Cl | H |
| 39 | 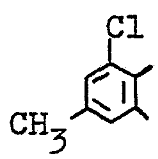 | " | 4-Cl |
| 40 | 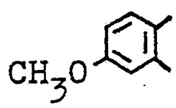 | " | " |
| 41 | " | " | 6-Cl |
| 42 | 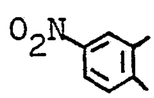 | " | " |
| 43 | 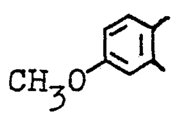 | 4-$CH_3$ | H |
| 44 | 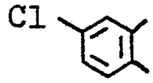 | " | H |

| Bsp. | 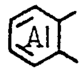 | $R^3$ | $R^4$ |
|---|---|---|---|
| 45 | 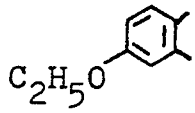 | $4-OCH_3$ | H |
| 46 | 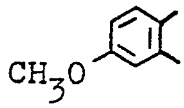 | " | H |
| 47 | " | $4-OC_2H_5$ | H |
| 48 | 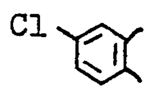 | " | H |
| 49 | 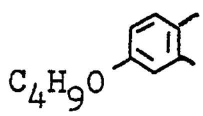 | $2-OCH_3$ | H |
| 50 | 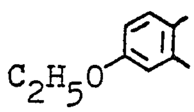 | " | H |
| 51 | 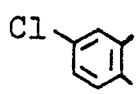 | 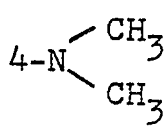 | H |
| 52 | 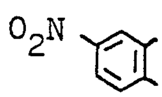 | $4-NO_2$ | H |
| 53 | 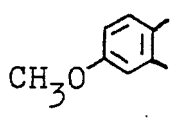 | " | H |

| Bsp. | 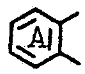 | $R^3$ | $R^4$ |
|---|---|---|---|
| 54 | 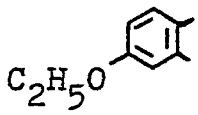 | 4-CN | H |
| 55 | 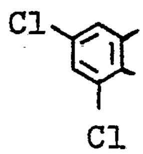 | " | H |
| 56 | 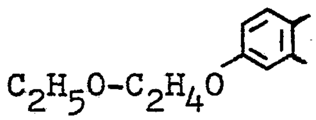 | $4-CO_2C_2H_5$ | H |

Beispiel 57

4,8 Teile der Verbindung der Formel

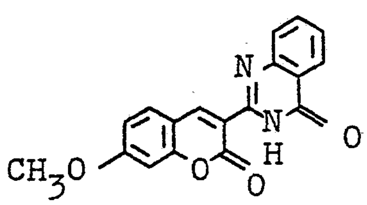

werden bei Raumtemperatur in 80 Volumenteilen Dimethylformamid mit 8,4 Teilen einer 6-n NaCN Lösung tropfenweise versetzt und 2 Stunden bei dieser Temperatur nachgerührt. Die Reaktionsmischung wird dann abgekühlt und

portionsweise bei 0 - 5 °C mit 15 Teilen Bleitetraacetat versetzt. Man rührt eine Stunde bei 0 - 5 °C nach und gießt das Reaktionsgemisch in Wasser. Der ausgefallene Farbstoff wird abgesaugt und mit viel Wasser gewaschen. Man erhält 5,8 Teile der Verbindung der Formel

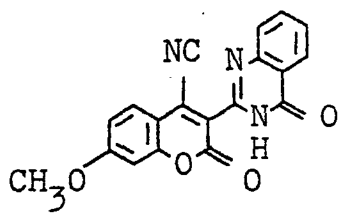

vom Schmelzpunkt 240 - 250 °C.

Analog lassen sich die in der Tabelle 3 aufgeführten Verbindungen herstellen.

0027529

Tabelle 3

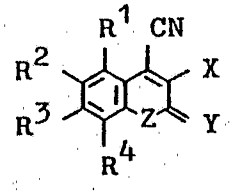

| Bsp. | $R^1$ | $R^2$ | $R^3$ | $R^4$ | X | Y | Z |
|---|---|---|---|---|---|---|---|
| 58 | H | H | $CH_3O-$ | H | 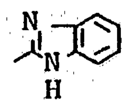 | O | O |
| 59 | " | " | " | " | 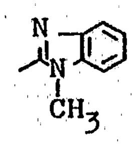 | " | " |
| 60 | " | " | " | " | 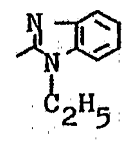 | " | " |
| 61 | " | " | " | " | 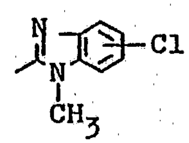 | " | " |

| Bsp. | $R^1$ | $R^2$ | $R^3$ | $R^4$ | X | Y | Z |
|---|---|---|---|---|---|---|---|
| 62 | H | H | $CH_3O-$ | H | 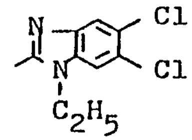 | O | O |
| 63 | " | " | " | " | 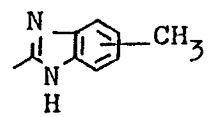 | " | " |
| 64 | " | " | " | " | 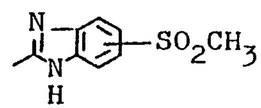 | " | " |
| 65 | " | " | " | " | 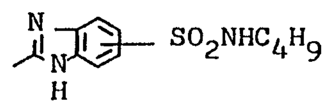 | " | " |
| 66 | " | " | " | " | 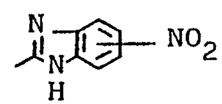 | " | " |
| 67 | " | " | " | " | 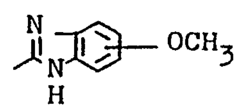 | " | " |

| Bsp. | $R^1$ | $R^2$ | $R^3$ | $R^4$ | X | Y | Z |
|---|---|---|---|---|---|---|---|
| 68 | H | Cl | $CH_3O-$ | H | 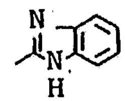 | O | O |
| 69 | " | " | H | Cl | 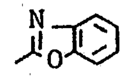 | " | " |
| 70 | " | " | " | " | 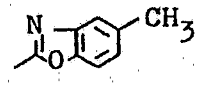 | " | " |
| 71 | " | " | $CH_3O-$ | " | " | " | " |
| 72 | " | H | $C_2H_5O-$ | " | " | " | " |
| 73 | " | " | $C_2H_5-OC_2H_4-O-$ | " | " | " | " |
| 74 | " | " | $CH_3O-$ | " | 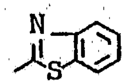 | " | " |
| 75 | " | " | " | " | " | $NC_6H_5$ | " |
| 76 | " | Cl | H | " | " | $NC_4H_9$ | " |

| Bsp. | $R^1$ | $R^2$ | $R^3$ | $R^4$ | X | Y | Z |
|---|---|---|---|---|---|---|---|
| 77 | H | Cl | H | H | 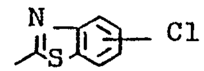 | O | O |
| 78 | " | " | " | " | 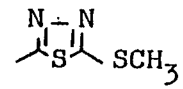 | " | " |
| 79 | " | " | $CH_3O-$ | " | 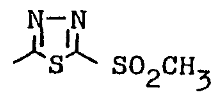 | " | " |
| 80 | " | " | $C_2H_5OC_2H_4O-$ | " | " | " | " |
| 81 | " | " | " | " | 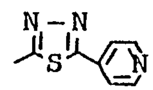 | " | " |
| 82 | " | " | $C_2H_5(OC_2H_4)_2-O-$ | " | 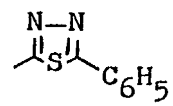 | " | " |
| 83 | " | " | $C_2H_5O-$ | " | 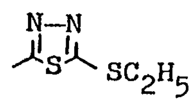 | " | " |
| 84 | " | " | " | " | 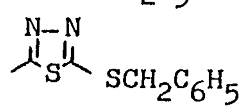 | " | " |

| Bsp. | $R^1$ | $R^2$ | $R^3$ | $R^4$ | X | Y | Z |
|---|---|---|---|---|---|---|---|
| 85 | H | Cl | $C_2H_5O-$ | H | 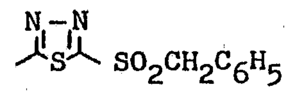 | O | O |
| 86 | " | " | $CH_3O-$ | " | 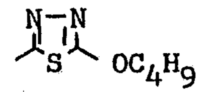 | " | " |
| 87 | " | " | " | " | 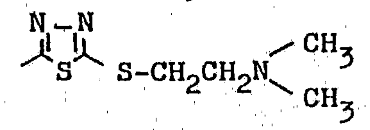 | " | " |
| 88 | " | " | " | " | 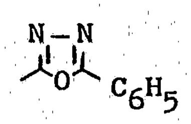 | " | " |
| 89 | " | " | " | " | 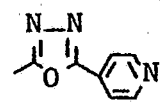 | " | " |
| 90 | " | " | " | " | 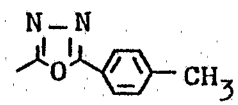 | " | " |
| 91 | " | H | $C_2H_5O-$ | " | 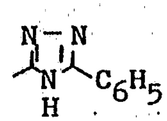 | " | " |
| 92 | " | " | " | " | 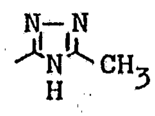 | H | " |

| Bsp. | $R^1$ | $R^2$ | $R^3$ | $R^4$ | X | Y | Z |
|---|---|---|---|---|---|---|---|
| 93 | H | H | $CH_3O-$ | H | 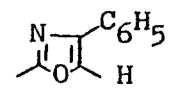 | H | O |
| 94 | " | " | " | " | 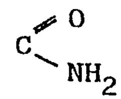 | " | " |
| 95 | " | " | " | " | 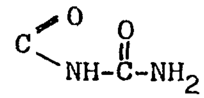 | " | " |
| 96 | " | " | " | " | 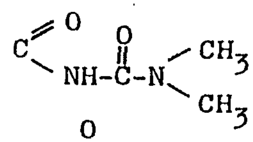 | " | " |
| 97 | " | " | $C_2H_5-O-$ | " | 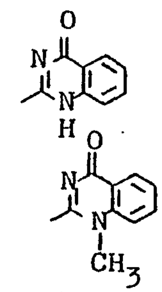 | " | " |
| 98 | " | " | $CH_3-O-$ | " |  | " | " |
| 99 | " | " | " | " | 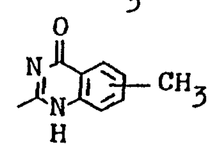 | " | " |

| Bsp. | $R^1$ | $R^2$ | $R^3$ | $R^4$ | X | Y | Z |
|---|---|---|---|---|---|---|---|
| 100 | H | H | $CH_3O-$ | H | $-SO_2C_6H_5$ | H | O |
| 101 | " | " | " | " | 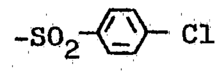 | " | " |
| 102 | " | " | " | " | 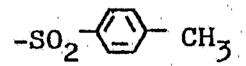 | " | " |
| 103 | " | " | " | " | 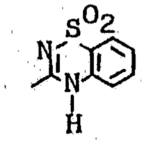 | " | " |
| 104 | " | " | " | " | 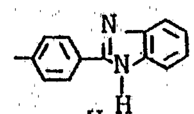 | " | " |
| 105 | " | " | " | " | 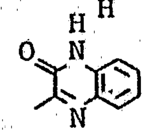 | " | " |
| 106 | " | " | " | " | 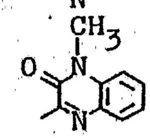 | " | " |

Beispiel 107

6 Teile der Verbindung

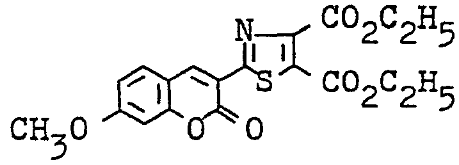

in 60 Volumenteilen Dimethylformamid werden mit 2,8 Teilen einer 6-n NaCN-Lösung tropfenweise versetzt. Durch leichtes Kühlen hält man die Temperatur unter 30 °C. Anschließend rührt man 90 Minuten bei 25 °C nach. Das Reaktionsgemisch wird dann auf 0 - 5 °C abgekühlt, vorsichtig mit 13,3 Teilen Bleitetraacetat versetzt und 1 Stunde bei 10 °C nachgerührt. Das Reaktionsgemisch wird in Eiswasser gegossen, abgesaugt und mit viel Wasser gewaschen. Man erhält 7,3 Teile der Verbindung

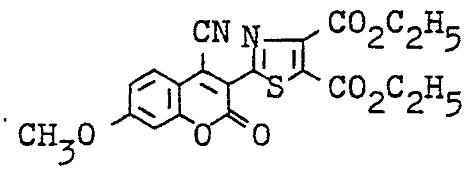

vom Schmelzpunkt 290 - 295 °C.

Die Ausgangsverbindung wird analog dem in der DE-OS 28 07 761 beschriebenen Verfahren aus 4-Methoxysalicylaldehyd und [4,5-Dicarbäthoxythiazolyl-2]-acetamid hergestellt.

[4,5-Dicarbäthoxy-thiazolyl-2]-acetamid wird analog dem in der DE-OS 28 07 761, Beispiele A - T, beschriebenen Verfahren aus Thiocarbamoylacetamid und Chloroxalessigsäurediäthylester hergestellt.

Analog lassen sich die in der Tabelle 4 aufgeführten Verbindungen synthetisieren.

Tabelle 4

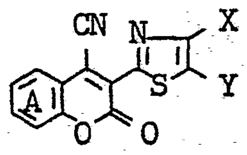

| Bsp. Nr. | 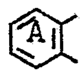 | X | Y |
|---|---|---|---|
| 108 | 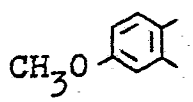 | $CO_2CH_3$ | $CO_2CH_3$ |
| 109 | " | $CO_2C_2H_5$ | $CO_2CH_3$ |
| 110 | " | $CO_2C_2H_4OC_2H_5$ | $CO_2C_2H_4OC_2H_5$ |
| 111 | " | $CO_2C_4H_9$ | $CO_2C_2H_5$ |
| 112 | 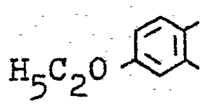 | $CO_3C_3H_7$ | $CO_2C_3H_7$ |

| Bsp. Nr. | 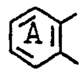 | X | Y |
|---|---|---|---|
| 113 | 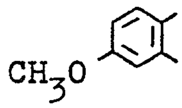 | $CO_2C_4H_9$ | $CO_2C_4H_9$ |
| 114 | " | $CO_2C_2H_4OC_6H_5$ | $CO_2C_2H_4OC_6H_5$ |
| 115 | " | $CO_2CH_2CH(C_2H_5)C_4H_9$ | $CO_2C_2H_5$ |
| 116 | 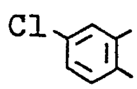 | $CO_2C_2H_5$ | $CO_2C_2H_5$ |
| 117 | " | $CO_2C_4H_9$ | $CO_2C_4H_9$ |

Beispiel 118

8,9 Teile der Verbindung aus Beispiel 107 werden in 50 Volumenteilen n-Butanol mit 0,5 Volumenteilen konzentrierter $H_2SO_4$ 8 Stunden unter Rückfluß gekocht. Das Reaktionsgemisch wird abgekühlt, abgesaugt und mit n-Butanol und Wasser gewaschen. Man erhält 8,5 Teile einer Verbindung, die identisch mit dem Farbstoff aus Beispiel 113 ist.

Beispiel 119

4,6 Teile 3-[4',5'-Dicyan-thiazolyl-2']-7-methoxycumarin in 40 Volumenteilen Dimethylformamid werden bei Raumtemperatur mit 3,3 Volumenteilen einer 6-n NaCN-Lösung versetzt. Das Reaktionsgemisch wird dann 1 Stunde bei 25 °C weitergerührt und anschließend auf 0 - 5 °C abgekühlt. Man gibt vorsichtig 8,8 Teile Bleitetraacetat zu und rührt 1 Stunde bei 5 °C nach. Die entstandene Suspension rührt man in Eiswasser ein. Das Reaktionsprodukt wird abgesaugt und mit Wasser gewaschen. Man erhält 5,7 Teile der Verbindung

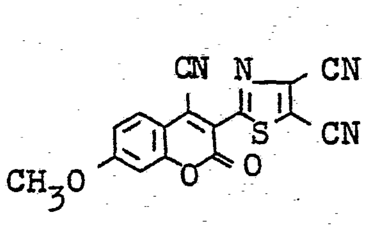

vom Schmelzpunkt 270 - 272 °C.

Die Ausgangsverbindung wurde wie folgt hergestellt:

10,3 Teile 3-[4',5'-Dicarbamoyl-thiazolyl-2']-7-methoxycumarin (aus [4,5-Dicarbamoyl-thiazolyl-2]-acetamid und 4-Methoxysalicylaldehyd nach bekannten Methoden hergestellt), werden in 50 Volumenteilen Dimethylformamid und 9 Volumenteilen Phosphoroxytrichlorid 30 Minuten bei 100 °C gehalten. Das Reaktionsgemisch wird dann in Eiswasser eingerührt, das ausgefallene Reaktionsprodukt nach 60 Minuten abgesaugt und mit Wasser neutral gewaschen.

Man erhält 8,6 Teile der Verbindung

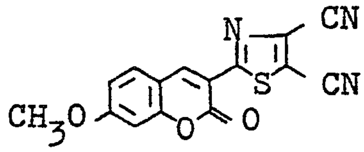

vom Schmelzpunkt 290 - 295 °C.

[4,5-Dicarbamoyl-thiazolyl-2]-acetamid kann aus [4,5-Dicarbäthoxy-thiazolyl-2]-acetamid und $NH_3$ hergestellt werden. Die Verbindung schmilzt bei 250 - 255 °C.

3-[4',5'-Dicyan-thiazolyl-2']-7-methoxycumarin kann auch aus 4-Methoxysalicylaldehyd und [4,5-Dicyanthiazolyl-2]-acetonitril nach bekannten Methoden synthetisiert werden.

Das [4,5-Dicyan-thiazolyl-2]-acetonitril kann aus [4,5-Dicarbamoyl-thiazolyl-2]-acetamid und Phosphoroxytrichlorid in indifferenten Lösungsmitteln hergestellt werden.

Analog kann man die in der Tabelle 5 aufgeführten Verbindungen herstellen.

Tabelle 5

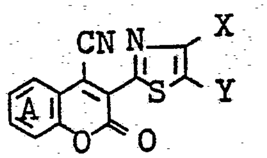

| Bsp. Nr. | 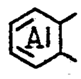 | X | Y |
|---|---|---|---|
| 120 | 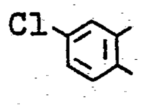 | $-CONH_2$ | $-CONH_2$ |
| 121 | " | CN | CN |
| 122 | 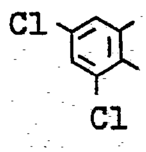 | $CONH_2$ | $CONH_2$ |
| 123 | " | CN | CN |
| 124 | 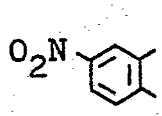 | $CONH_2$ | $CONH_2$ |
| 125 | " | CN | CN |
| 126 | 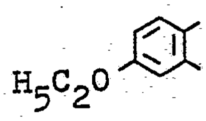 | $CONH_2$ | $CONH_2$ |
| 127 | " | CN | CN |
| 128 | 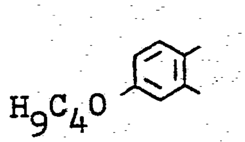 | $CONH_2$ | $CONH_2$ |
| 129 | " | CN | CN |

Patentansprüche

1. Heterocyclische Verbindungen der allgemeinen Formel

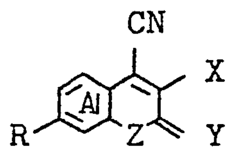 I ,

in der

R Wasserstoff, gegebenenfalls substituiertes Alkyl, Cycloalkyl, Alkoxy, Phenoxy, Hetaryloxy, Alkylmercapto, Arylmercapto, Aryl, Acylamino, Hydrazino, Hetarylamino, Acyloxy, Alkylaminocarbonyloxy oder Arylaminocarbonyloxy, Halogen, Hydroxyl, Nitro, Sulfonamido, Cyan oder einen in o-Stellung ankondensierten Ring,

X einen elektronenziehenden Rest,

Y NH, $NR^1$, O, S oder 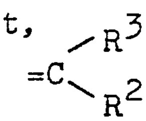 und

Z O, NH, $NR^1$ oder S bedeuten, wobei

$R^1$ gegebenenfalls substituiertes Alkyl, Cycloalkyl, Aryl oder Acyl und

$R^2$ und $R^3$ unabhängig voneinander Cyan, Carbonester, gegebenenfalls substituiertes Carbamoyl oder Hetaryl sind und wobei der Ring A noch durch einen weiteren Rest R substituiert sein kann.

2. Verbindungen der Formel gemäß Anspruch 1, bei denen

R Alkyl, Alkoxy, Acylamino, Acyloxy oder Alkylaminocarbonyloxy mit jeweils 1 bis 4 C-Atomen oder Triazinyloxy- und

X einen Rest der Formel

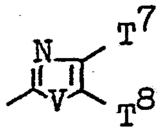 oder 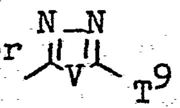

bedeuten, wobei

V ein Rest der Formel -S-, -O-, -NH oder $N-T^{10}$,

$T^7$ und $T^8$ unabhängig voneinander Wasserstoff, gegebenenfalls substituiertes Alkyl oder Phenyl, gegebenenfalls substituiertes Alkyl- oder Phenylmercapto, Cyan, Carbamoyl oder Carbonester oder ggf. im Ring durch Chlor, Brom, Methyl, Äthyl, Methoxy oder Äthoxy substituiertes Benzyl,

$T^8$ darüber hinaus noch $C_2$ - $C_4$-Alkanoyl, gegebenenfalls durch Chlor, Methyl, Methoxy oder Äthoxy substituiertes Benzoyl, $C_1$- bis $C_6$-Alkylsulfonyl oder gegebenenfalls durch Chlor, Brom, Methyl, Methoxy, Äthoxy, Amino, $C_1$ - $C_4$ Alkanoylamino oder Benzoylamino substituiertes Phenylsulfonyl,

$T^7$ und $T^8$ zusammen einen gesättigten, gegebenenfalls Sauerstoff oder Schwefel enthaltenden 5- oder 6-gliedrigen gesättigten Ring oder einen gegebenenfalls ein- oder mehrfach durch $C_1$ - $C_4$ Alkyl, Phenyl, Chlor, Brom, Alkoxy oder Alkylsulfonyl substituierten Benzring,

$T^9$ gegebenenfalls substituiertes Alkyl, Alkenyl, Cycloalkyl, Aralkyl, Aryl oder Heteroaryl oder ein Rest der Formel O-B, -SB, $-SO_2B$ oder

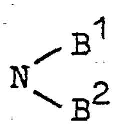 und

$T^{10}$ gegebenenfalls substituiertes Alkyl oder Aryl sind, wobei

B einen gegebenenfalls substituierten Alkyl-, Alkenyl-, Cycloalkyl-, Aralkyl-, Aryl oder Heteroarylrest

$B^1$ und $B^2$ unabhängig voneinander Wasserstoff, gegebenenfalls substituiertes Alkyl, Cycloalkyl, Aralkyl oder Aryl oder

$B^1$ und $B^2$ zusammen mit dem Stickstoff einen 5- oder 6-gliedrigen heterocyclischen Ring bedeuten.

3. Verbindungen gemäß Anspruch 1 der allgemeinen Formel

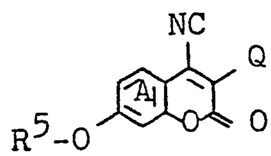 ,

bei denen

$R^5$ H, $C_1$ - $C_4$-Alkyl, $C_1$ - $C_{14}$-Phenoxy- oder Alkoxypolyalkoxyalkyl, durch Chlor, Brom, Methyl, Äthyl, Methoxy oder Äthoxy substituiertes Phenyl oder $C_1$ - $C_6$-Acyl und

Q einen Rest der Formel

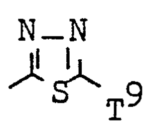

oder

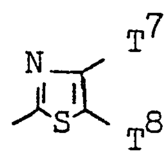

bedeuten, wobei

$T^7$, $T^8$ und $T^9$ die bereits angegebene Bedeutung besitzen.

4. Die Verwendung der Verbindungen gemäß Anspruch 1 zum Färben synthetischer Fasern oder von Kunststoffen.

0027529

Europäisches Patentamt

# EUROPÄISCHER RECHERCHENBERICHT

Nummer der Anmeldung
EP 80 10 5269.7

## EINSCHLÄGIGE DOKUMENTE

| Kategorie | Kennzeichnung des Dokuments mit Angabe, soweit erforderlich, der maßgeblichen Teile | betrifft Anspruch |
| --- | --- | --- |
| X | DE - A1 - 2 844 299 (CIBA-GEIGY)<br>* Ansprüche 1, 38 *<br>-- | 1,4 |
| D | DE - A1 - 2 807 761 (BASF)<br>* Ansprüche 1, 4 *<br>-- | 1,4 |
| | DE - B1 - 2 739 841 (BAYER)<br>* Anspruch 1 *<br>---- | 4 |

KLASSIFIKATION DER ANMELDUNG (Int.Cl.³)

C 07 D 417/04
C 07 D 311/16
C 07 D 405/04
C 09 B 57/02
C 08 K 5/47

RECHERCHIERTE SACHGEBIETE (Int. Cl.³)

C 07 D 311/16
C 07 D 417/04
C 08 K 5/47
C 09 B 57/02

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
| --- | --- | --- |
| Berlin | 13-01-1981 | FROELICH |